# EUROPEAN PATENT SPECIFICATION

(11) **EP 2 079 681 B1**
(45) Date of publication and mention of the grant of the patent: **15.09.2010**
(21) Application number: 07824449.8
(22) Date of filing: 05.11.2007
(51) Int. Cl.: C07C 233/63, A61K 31/223, A61P 11/02, A61P 11/04, A61P 11/14

(54) **N-ALKYLCARBONYL-D-AMINO HYDROXYALKYL ESTER COMPOUNDS AND THEIR USE**
N-ALKYLCARBONYL-D-AMINOHYDROXYALKYLESTERVERBINDUNGEN UND IHRE VERWENDUNG
COMPOSÉS D'ESTER N-ALKYLCARBONYL-D-AMINO HYDROXYALKYLE ET UTILISATION DE CES DERNIERS

(30) Priority: 08.11.2006 US 857897 P; 02.04.2007 US 921513 P
(43) Date of publication of application: 22.07.2009
(73) Proprietor: Wei, Edward T., Berkeley, CA 94708 (US)
(72) Inventor: Wei, Edward T., Berkeley, CA 94708 (US)
(74) Representative: Florence, Julia Anne
(86) International application number: PCT/GB2007/004213
(87) International publication number: WO 2008/056119

(56) References cited:
- WO-A-2006/103401
- GB-A- 1 351 761
- US-A1- 2007 155 755

## Description

### RELATED APPLICATIONS

### TECHNICAL FIELD

The present invention pertains to certain *N*-alkylcarbonyl-D-amino acid hydroxylalkyl esters (NACHE), including, for example, (*R*)-2-[((1*R*,2*S*,5*R*)-2-isopropyl-5-methyl-cyclohexanecarbonyl)-amino]-propionic acid 3-hydroxy-propyl ester, and compositions comprising such compounds, that target sensory elements on nerve fibers and which are usefully administered to refresh and cool the skin and mucous membranes. This disclosure generally relates to menthol-like cooling compounds, which do not have the smell, taste, or short duration of action of menthol. These compounds are designed for delivery onto the surfaces of the oral cavity, upper respiratory tract and skin. These compositions produce refreshing and cooling sensations and are expected to inhibit the perception of cough stimuli, itch, pain, and discomfort from the body's surfaces. In the oral cavity these compositions produce refreshing cooling without adverse effects on taste. These compounds have special utility for mouth refreshment and for the treatment of respiratory disorders such as cough, asthma, and chronic obstructive pulmonary diseases. In addition, topical use inhibits perception of itch, irritation, pain and discomfort.

### BACKGROUND

A number of patents and publications are cited herein in order to more fully describe and disclose the invention and the state of the art to which the invention pertains.

Throughout this specification, including the claims which follow, unless the context requires otherwise, the word "comprise," and variations such as "comprises" and "comprising," will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Thus, for example, reference to "a pharmaceutical carrier" includes mixtures of two or more such carriers, and the like.

Ranges are often expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment.

This disclosure includes information that may be useful in understanding the present invention. It is not an admission that any of the information provided herein is prior art or relevant to the presently claimed invention, or that any publication specifically or implicitly referenced is prior art.

About three decades ago, a group of scientists synthesized over 1200 compounds in an attempt to find cooling agents that had properties better than menthol. Their results were summarized in a paper (Watson et al., 1978, "New compounds with the menthol cooling effect," J. Soc. Cosmet. Chem., Vol. 29, pp. 185-200). From this research, an *N*-alkylcycloalkyl- and an *N*-alkyl-alkyl carboxamide, WS-3 and WS-23, reached the market and are used as additives to confectionery, comestibles (e.g., candy, chewing gum), and toiletries. U.S. Patent 4,178,459 (Watson et al., December 11, 1979) described cooling properties of some *N*-alkoxycarbonylalkyl-substituted p-methane-carboxamides. Other menthol-like cooling compounds in commercial use for applications to skin and mucous membranes are, for example, menthyl lactate (Frescolate ML) and menthoxypropanediol (Cooling Agent 10). The recent information on cooling agents used for topical applications has been reviewed (see, e.g., Erman, M.B., May 2005, "Cooling agents and skin care applications", Cosmetics & Toiletries, Vol. 120, pp. 105-118; Erman, M.B., 2004, "Progress in physiological cooling agents", Perfumer & Flavorist, Vol. 29, pp. 34-50; Jacobs, P. and Johncock, W., 1999, "Some like it cool", Parfumerie und Ksometik, Vol. 80, pp. 26-31). Cooling compounds are described in U.S. Patent 6,919,348 (Wei et al., July 19, 2005). Other molecules investigated by Wei are described in: US 2005/0059639, published March 17, 2005, Ophthalmic Compositions and Methods for Treating Eye Discomfort and Pain; US 2005/0159394, published July 21, 2005, Aryl-Substituted Derivatives of Cycloalkyl and Branched Chain Alkyl Carboxamides and Carboxylic Acids Useful as Antinociceptive Drugs For Peripheral Targets;

US 2005/0187211, published Aug. 25, 2005, *N*-Aryls-Carboxamide Compositions and Methods; and WO 2006/103401, *N*-Alkylcarbonyl-Amino Acid Ester and *N*-Alkylcarbonyl-Amino Lactone Compounds and Their Use, published Oct. 5, 2006. GB 1351761 describes N-substituted-p-menthane-3-carboxamides having a physiological cooling effect on the skin and on the mucous membranes of the body.

### BRIEF DESCRIPTION OF THE DRAWINGS

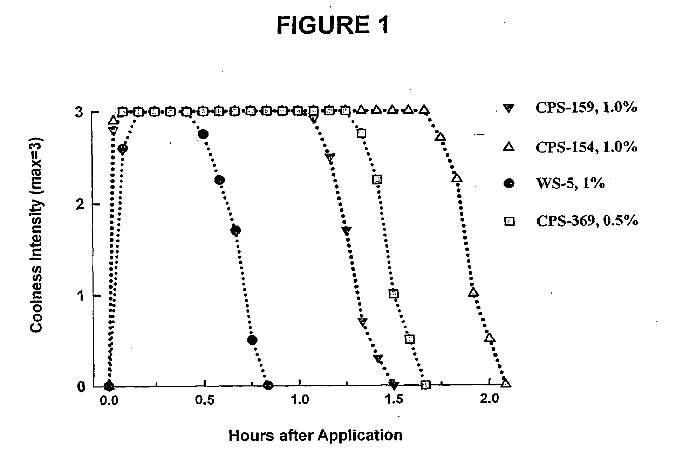

Figure 1 shows a graph of coolness intensity as a function of time after application, for four compounds, CPS-159, CPS-154, WS-5, and CPS-369, as described in Example 1.

### SUMMARY OF THE INVENTION

The present invention relates to the discovery that certain *N*-alkylcarbonyl-D-amino acid hydroxylalkyl esters (NACHE), described herein, have the desirable quality of pleasant cooling action when applied to the facial skin. The duration of action of these compounds exceeds one hour and occurs with minimal or no eye irritation, tingling, smarting, or burning sensations of the skin. In therapeutic situations where prolonged relief of sensory discomfort is desired NACHE may be applied to skin to counteract irritation, itch and pain. Some of the NACHE, when put into the mouth, produce rapid onset of refreshing sensations of coolness and, surprisingly, are complete devoid of taste. The hydroxyalkyl ester function confers increased aqueous/alcohol solubility of these compounds and, hence, these compounds are ideally suited for use in comestibles (things that are put into the mouth), beverages, and confectionery. Another ideal use is for upper respiratory disorders such as cough, where the cooling sensations of NACHE relieve throat irritation, and the active ingredient does not have unpleasant taste.

Preferred compounds are chemical entities known as *N*-cycloalkylcarbonyl D-amino acid hydroxylalkyl esters or *N*,*N*-dialkylcycloalkyl carbonyl D-amino acid hydroxylalkyl esters if a methyl group is added to the amido nitrogen. These compounds are collectively abbreviated as NACHE. The preferred orientation of the α-carbon is in the D-configuration (or *R*-configuration, using the the Cahn-Ingold-Prelog nomenclature system). The D-configuration has the effect of increasing potency by prolonging drug action, and of producing a selective refreshing coolness. The NACHE are particularly preferred embodiments because of improved aqueous/alcohol solubility and an absence of unpleasant taste. Especially preferred is where the cycloalkyl group is p-menthane, the amino acid is D-alanyl, and the ester is the hydroxyethyl or hydroxypropyl ester.

One aspect of the present invention pertains to certain compounds, specifically, compounds having the structure of Formula 1: 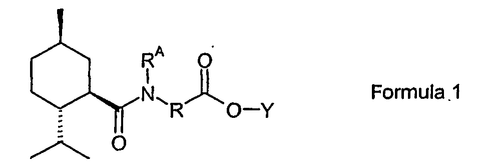 wherein:
R^{A} is -H or -CH₃;
R is a branched C₂-C₄ alkylidene, with the α-carbon in the D-configuration; and
Y is C₂-C₅ hydroxyalkyl.

In one embodiment, the compound is (*R*)-2-[((1*R*,2*S*,5*R*)-2-isopropyl-5-methyl-cyclohexanecarbonyl)-amino]-propionic acid 3-hydroxy-propyl ester.

In one embodiment, the compound is (*R*)-2-[((1*R*,2*S*,5*R*)-2-isopropyl-5-methyl-cyclohexanecarbonyl)-amino]-propionic acid 2-hydroxy-1-methyl-propyl ester.

One aspect of the present invention pertains to compositions comprising such compounds.

One aspect of the present invention pertains to such compounds and compositions for use in a method of treatment of the human or animal body by therapy; for use in a method of treatment of a respiratory disorder, an upper respiratory disorder, cough, asthma, or a chronic pulmonary disease; for use in a method of treatment of cough or throat irritation; for use as an anti-irritant.

One aspect of the present invention pertains to use of such compounds and compositions in the manufacture of a medicament for the treatment of a respiratory disorder, an upper respiratory disorder, cough, asthma, or a chronic pulmonary disease; for the treatment of airway irritation or obstruction; for the treatment of cough or throat irritation; for inhibiting the perception of itch, irritation, pain and discomfort; for use as a local analgesic on inflamed skin; for use as an anti-pruritic; for use as an anti-irritant.

### DETAILED DESCRIPTION OF THE INVENTION

The inventor has identified, by experiment, a set of compounds, shown in Formula 1, that has the following properties:
- a cooling and refreshing effect on the skin and mucous membranes, that in the presence of tissue injury or inflammation, have anti-irritancy, anti-pruritic, and anti-nociceptive effects that are readily obtained on surfaces such as the periorbital skin, the cheeks (malar region) and the lips and skin above the upper lip, and in the oral cavity and throat.
- a minimal irritant action on the eye when the compound is applied to facial skin about the eyes.
- a duration of action that exceeds 1 hour when applied on the skin at a concentration of 40 mM or less, equivalent to a ∼1% wt/vol mixture.
- repeat applications do not result in altered sensitivity to subsequent stimulation.
- when applied into the oral cavity, a cool and refreshing sensation occurs without odor or taste.
- when tested in standardized pharmacokinetic models (KnowitAll Informatics^{™}, from BioRad Laboratories, Richmond, California, or ADMET Predictor^{™} and Modeler^{™} from Simulation-Plus, Lancaster, California), based on *in silico* simulation, these compound are soluble in ethanol and have aqueous solubility in excess of 0.65 mol/L.

The compounds are compounds of Formula 1: 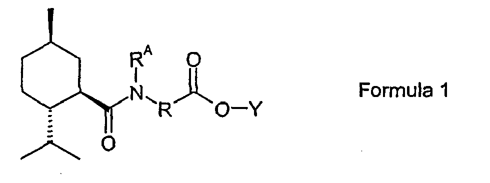 wherein:
R^{A} is -H or -CH₃;
R is a branched C₂-C₄ alkylidene, with the α-carbon in the D-configuration; and
Y is C₂-C₅ hydroxyalkyl.

The term "alkylidene" means a straight or branched saturated, aliphatic, divalent radical having the number of carbon atoms indicated. In the structures of Formula 1, if R is equal to 2 or greater, the α-carbon atom has a chiral center. Of the two enantiomers, the D-configuration is preferred over the L-form. Overall, the structure may be viewed as a *N*-acylated-D-amino acid hydroxyalkyl ester. For the p-menthoyl moiety the preferred stereoisomer (one of eight possibilities) is for the p-menthane to be in the I-menthol configuration.

Examples of compounds of Formula 1 are:

| Name | Structure |
|---|---|
| (*R*)-2-[((1*R*,2*S*,5*R*)-2-isopropyl-5-methyl-cyclohexanecarbonyl)-amino]-propionic acid 3-hydroxy-propyl ester. | 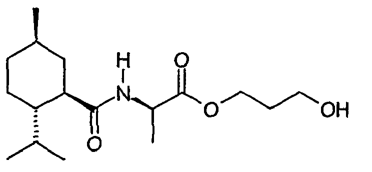 |
| (*R*)-2-[((1*R*,2*S*,5*R*)-2-isopropyl-5-methyl-cyclohexanecarbonyl)-amino]-propionic acid 2-hydroxy-1-methyl-propyl ester | 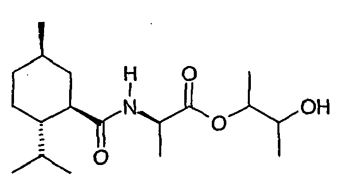 |

### Pharmacology of NACHE

Cooling of the skin and mucous membranes is detected by a subset of primary sensory afferents that have receptors on nerve endings. These sensory fibers exhibit a rhythmic, ongoing discharge at neutral temperatures that increases in response to skin temperature reductions (from 33°C to 23°C) and is suppressed by warming. The dynamic information is propagated along axons in spike trains, at about 20 to 40 impulses/sec, to central neurons, leading in humans to cooling sensations. This type of sensation is mimicked by facial exposure to temperatures of 15°C to 22°C.

Temperature detection inputs from the face, lips and oral cavity are especially important for modulating behavior as this surface is densely innervated, as can be seen in diagrams of the sensory homunculus in textbooks of psychology. This fact is readily experienced as we notice temperature changes easily from sensations on our face but less so from other parts of our body. The three branches of the trigeminal nerve - ophthalmic, maxillary, and mandibular - send afferent thermosensitive information from the cutaneous surfaces of the face, head, scalp, lips, nasal and oral membranes, and dorsal surface of the tongue, to surface nuclei of the brainstem. Cold receptor signals from these sites, representing 95% of the tonic neuronal discharges, dominate input, as warmth/heat detection is not tonically active. Other important cooling signals from the pharynx are propagated along the glossopharyngeal nerve.

Sensations can be "confusing" when a chemical affects more than one sensory modality. This is especially true for (-)-menthol (synonyms: I-menthol, (1*R*)-menthol, (1*R*,2*S*,5*R*)-menthol). (-)-Menthol is widely used as a cooling agent but it has multimodal action on sensory processes. For example, in the upper airways and oral cavity, (-)-menthol can elicit somatosensation (cooling, irritation, tingling), olfaction (minty), and gustation (bitter). As a counter-irritant, (-)-menthol can reduce irritation of oral and pharyngeal membranes (e.g., strong mints or toothpastes) and have analgesic actions on muscle (e.g., BenGay® ointment). The multimodalities may further mix to give rise to complex perceptions of irritation (burning, prickling, stinging), especially around the eyes, of thermal effects (cooling, warming) and of tactile effects (buzzing, tingling, tickling, numbing). In the nose and oral cavity, the predominant mode of detecting (-)-menthol is olfactory (Nagata et al., 2005, J. Exptl. Psychol., Vol. 31, pp. 101-109).

The multimodal action of (-)-menthol and related agents on sensory processes are utilized in compositions for food, confectionery, flavors, chewing gum, mouth fresheners, liptsticks, and other comestibles (items put in the mouth), beverages, tobacco products, toiletries, over-the-counter pharmaceutical compositions for treatment of nasal and airway symptoms, for gastrointestinal tract distress, and as a counter-irritant for alleviating discomforts of skin and muscle. Menthol confectionery also has alerting effects on the central nervous system.

In certain applications, it is desirable to have molecules that retain the refreshing and cooling effects of menthol but without irritation, taste or odor. Such molecules like NACHE can be used to refresh or to relieve sensations of irritation, itch and pain.

### Bioassays of NACHE

Psychic events such as cooling, refreshment, relief of irritation, itch, and pain, cannot be expressed by animals. Receptor assays, based on cells transfected with the genes for proteins associated with thermosensation (e.g., TRP-M8 or TRP-A1) may be used as a model of sensory processes. The receptor assays yield quantitative data, but these assays give no information on onset and offset of action, or on the quality of human sensations evoked by the chemicals. Thus, the best information on the pharmacological properties of chemicals is derived from direct tests on humans.

Rowsell et al. (US Pat. 4,178,459) tested the properties of *N*-substituted p-menthane carboxamides on volunteers by putting filter paper (1 x 1 cm), impregnated with a known amount of compound, onto the dorsal surface of the tongue of the test subject. After 30 seconds, the subject was required to report presence or absence of a cooling effect. These data were reported as "Threshold, µg" and refer to the threshold amount of the test substance that produces cooling sensations upon application onto the tongue of a panel of human volunteers. The average threshold of (-)-menthol for 6 subjects was 0.25 µg, but there was a 100-fold variation in individual sensitivity.

The inventor has found that the cooling and sensory properties of a NACHE can be tested by dissolving a test substance in an ointment (usually Aquaphor® which is 41 % petrolatum, and the rest mineral oil, ceresin and lanolin alcohol) and singly applying the ointment (40 to 70 mg) onto the skin surface using a plastic stick. A reliable place for topical application is the skin above the upper lip (above the vermilion border of the lips), on the philtrum, lateral to the philtrum until the nasolabial folds, and on the lower nostrils (subnasale). This part of the face is known to be densely innervated with cold receptors, second only to the surfaces of the eyeball and anogenitalia. A second location is on or below the malar eminence (cheekbone). The skin above the cheekbone is thicker than above the lips, and therefore has a higher threshold for activation. At both loci, tingling, cool and cold sensations in the skin may be experienced and rated for time of onset and intensity.

The intensity of the subjective skin sensation is rated as 0, 1, 2 or 3 with 0 as no change, 1 as slight coolness, cold, or tingling, 2 as clear-cut signal of coolness, cold, or tingling, and 3 as robust cooling or cold. The intervals for recording sensations are 5 to 10 minutes, until two successive zeroes are obtained. The results (shown in Figure 1) are averaged values of 4 to 6 separate trials in the same individual. The data are plotted using SigmaPlot (Systat Software, Point Richmond CA) and a smoothing function with a negative eponential was used for analysis and statistical fit of the results. The onset of drug action is taken as the time to reach 2 units of coolness intensity, and offset of drug action is the time when coolness intensity drops below 2, after previously surpassing 2 units. The duration of cooling action is defined as the offset time minus the onset time.

As shown in the Examples, the NACHE preferred embodiments, tested at 40 mM, produce cooling sensations on the facial skin, have a rapid onset of action (less than 5 minutes) and slow offset (more than 1 hour). The long duration of action of CPS-154 illustrates the potent activity of the D-amino acid derivative of alanine.

The NACHE were also tested in the oral cavity using these methods: a) placement of 2 mg of the crystalline chemical on the dorsal surface of the tongue, b) incorporation of 2 mg of NACHE into a rapidly-distintegrating tablet of 60 mg weight, and placement of the tablet on the caudal-dorsal surface of the tongue, c) placement of a 0.3 mL aliquot of a 5 mg/mL solution of a NACHE in a 50% ethanol-distilled water solution, and d) delivery of a 5 mg/mL solution of a NACHE in a 50% ethanol-distilled water solution into the oral cavity using a hand-activated mouth spray dispenser. The tests in the oral cavity were important because many cooling agents used in confectionery and comestibles have disagreeable tastes of bitterness, harshness or burning qualities that limit their utility in such items as candy, mints, mouthwashes, mouth fresheners, and cough syrups.

Surprisingly and unexpectedly, the NACHE tested were devoid of any unpleasant taste, yet retained robust refreshing cooling actions and were able to suppress throat irritation and cough. Additionally, it was noted that the presence of the hydroxyalkyl ester group increased solubility, which is desirable for drug delivery because many non-aqueous solvents also impart unpleasant tastes.

To the inventor's knowledge, the NACHE have never been synthesized and tested in the oral cavity. It was not previously known that the presence of a hydroxyl group on the alkyl ester of the parent molecule is associated with an absence of unpleasant taste, yet retaining the robust cooling action. Furthermore, these compounds have not been formulated into rapidly disintegrating tablets for the treatment of cough and throat irritation.

### Topical Uses of NACHE

In the best mode contemplated for practicing this invention, a NACHE embodiment is topically applied to relieve the irritation, itch, and pain of injured or inflamed tissues. Another contemplated use is as ingredients in comestibles (e.g., chewing gum, mouthwashes, anti-gingivitis products), and especially as a refreshing agent in mouth-fresheners.

By "topically" is meant application onto surfaces of the body in contact with air, which includes the skin, the eye surface, the lips, the upper (nasal membranes and pharyngeal surfaces) and lower respiratory tracts, and the entrance and exit of the gastrointestinal tract, that is, the oral cavity and the anorectum. Particularly favored sites of application are the surfaces innervated by the trigeminal and glossopharyngeal nerves which includes the facial skin, orbit, lips, nasal and oral cavities and the throat.

Some of the uses may be categorized as:
- therapeutic: a NACHE may, for example, be used as a local analgesic on inflamed skin or as an anti-pruritic.
- therapeutic: a NACHE may be incorporated into a tablet, lozenge, pastille, troche, mouth spray, syrup, or chewing gum to relieve throat irritation or cough.
- therapeutic: a NACHE may be incorporated into a tablet, mouth spray, nebulized aerosol, or syrup for treatment of obstructive pulmonary disorders such as nasal congestion, snoring, sleep apnea, cough, dyspnea, asthma or chronic obstructive pulmonary disease.
- anti-irritant: a NACHE may be incorporated into a skin care product to counter-act other irritating substances, such as retinoids or α- or ω-fatty acids.
- arousal: in normal, healthy individuals, the NACHE compositions described here may be used in a towel or towelette to alert and to refresh, to counteract fatigue, and

to relieve the individual from heat exhaustion, nasal and eye irritation. It may be used to enhance a bright-eyed and alert look because of its refreshing properties.
- cleansing: a NACHE may be incorporated into a towelette for removing make-up, especially for mascara around the eyes.
- food and personal care products: a NACHE may be incorporated into comestibles (e.g., chewing gum), cosmetics, lipsticks, flavors, confectionery, tobacco, beverages, or toiletries, to provide sensory refreshment.

Therapeutic uses for topical formulations of one or more NACHE embodiment(s) are contemplated in a towelette, lotion, cream, ointment, in aerosolized formulations, or in oral formulations, and include utility for a) alleviation of irritation, itch and pain from various forms of dermatitis (atopic, contact and irritant); b) pain from burned, traumatized or irritated skin (e.g., laser surgery), and from procedures related to wound debridement; c) itch and discomfort from skin infections, insect bites, sunburn, photodynamic treatment of skin (e.g., actinic keratoses, basal cell carcinoma); d) pruritus due to xerosis; e) muositis, stomatitis, cheilitis or itching of the lips from cold sores and gingivitis; f) pruritus ani, hemorrhoidal discomfort, pain from anal fissures, pain or itch from anal fistulas, pain from hemorrhoidectomy, perineal inflammation, anogenital skin inflammation and discomfort due to various local causes such as incontinence, diaper rashes, perineal inflammation; g) vulval pruritus and pain (e.g., from candidiasis or idiopathic, such as vulva vestibulitis and vulvodynia), dyspareunia, anogenital infections, including warts and sexually transmitted diseases, viral infections of the skin (especially in immunocompromised patients); h) nostril and nasal or upper airway discomfort from breathing obstruction, e.g., congestion, rhinitis, asthma, bronchitis, emphysema and chronic obstructive pulmonary diseases, dyspnea, sleep apnea and snoring; and i) conjunctivitis, ocular surface irritation, pain from corneal abrasions, and pain from eye surgery.

As sensory processes are also important in hollow viscus, the long-acting NACHE embodiments may be delivered nasally, or inhaled or encapsulated for oral delivery to the surfaces of the gastrointestinal tract, the urinary bladder, and the airways. For the lower gastrointestinal tract, the long-acting NACHE may be used to relieve heartburn, peptic pain, and the discomforts of irritable bowel and inflammatory bowel diseases. The preferred method of delivery would be enteric-coated capsules. Alternatively, a NACHE may be extruded onto the gut surface using a controlled release device such as an osmotic mini-pump. For the urinary bladder surface, an oral formulation of a NACHE may be used to suppress the discomforts of interstitial cystitis and relieve the symptoms of overactive bladder. For the upper airways, long-acting NACHE, inhaled or delivered as a mist or spray, may mimic (-)-menthol and relieve congestion, relax bronchial smooth muscles, and symptomatically relieve the choking sensations of dyspnea.

### Delivery to Target of NACHE

The access and residence of the agonist molecule at the receptor sites in an important factor for drug action. A key pharmacokinetic determinant of drug distribution and delivery on the skin is the octanol/water partition coefficient. Thus, the logarithm of the octanol/water partition coefficient (log P) in the range of 2.0 to 4.0 is considered ideal for activation of cool receptors in the skin. More ideally, the range is 2.5 to 3.5, because it is not desirable for the drug to cross the blood-brain-barrier (BBB) if it is absorbed into the systemic circulation. Sophisticated ADMET analysis programs, such as are available from Simulation-Plus (Lancaster, California) can predict drug passage across the BBB, based on the parameters described in Crivori et al., 2000, "Predicting blood-brain barrier pemeation from molecular structure" J. Med. Chem., Vol. 43, pp. 2204-2216.

An analysis of the structure-activity relationships from the present studies indicates that the presence of a hydroxyalklyl group on the ester, preferably hydroxyethyl or hydroxypropyl, of *N*-substituted-p-menthane-D-amino acid esters confer the desirable quality of low penetration across the BBB yet retaining adequate cooling properties. The presence of polar entities on the molecule in the addition to the hydroxyalkyl group, contributes to desirable ADME (absorption, distribution, metabolism, excretion) properties that will permit use on the skin and oral and respiratory membranes, and, if absorbed, has reduced risk of penetration of the BBB. This analysis of ADME properties adds a feature of novelty in the design of the NACHE and was determined by modelling programs designed to simulate pharmacokinetic parameters of ideal drug candidates.

In formulating topical compositions to practice this discovery, the NACHE may be incorporated into a vehicle that by itself may be inert or may contain other active ingredients. Suitable topical formulations, for example, include compositions such as liquids, aerosols, powders, pastes, lotions, liniments, creams and ointments, and cosmetic preparations. A wide variety of vehicles will be suitable, depending upon the particular product involved, such vehicles including solids, liquids, emulsions, foams and gels. Typical vehicles include oils and fats such as hydrocarbon oils, fatty acid esters, long chain alcohols, polyhydric alcohols, and silicone oils; finely divided solids such as starch or talc; low-boiling hydrocarbons; gums and natural or synthetic resins.

Of particular utility are suitable formulations for the oral cavity and throat, for example, compositions such as orally-disintegrating tablets, lozenges (cough drops, troches or pastilles), and mouth sprays. A typical tablet/lozenge is composed predominantly of an inert vehicle, carrier, or diluent. A medicinal agent is interspersed within this carrier. The tablet/lozenge will dissolve when placed in the oral cavity thereby releasing the medicinal agent so that it may come in contact with the tissues of the mouth and throat. The difference between a tablet and a lozenge mainly is size and rate of dissolution. A typical diluent, carrier, or vehicle may be a "polyhydric alcohol" construed as describing the following substances: xylitol, mannitol, sorbitol, maltitol, isomaltitol, maltotriitol, lactitol, and β-linked-glucopyranasido-sorbitol. Flavoring agents such as the sweeteners (e.g., aspartame, sucralose, or saccharin), berry flavors, or chocolate, may be added to mask any undesirable tastes. The mix is granulated to a uniformly dispersed blend, dispersing agents, anti-caking agents, and lubricants may be added and the mixture is then compressed to form tablets for oral administration. Alternatively, the active ingredient may be put in a chewing gum formulation. These methods are familiar to those skilled in art and are, for example, described in US Patent 5,322,694 (David Sixsmith, Pharmaceutical Lozenges) and in US Patent 5,846,557 (Eisenstadt et al., Chewing gum containing cough suppressing agent), both of which are incorporated herein by reference.

The duration of action of the active preparation may be further enhanced by the incorporation of mucoadhesive agent. Such mucoadhesives are, for example, described in US Patent 6,638,521 (D.J. Dobrozsi, Oral liquid mucoadhesive compositions) and in US Patent 6,562,363 (J. Mantelle et al., Bioadhesive compositions and methods for topical administration of active agents), both of which are incorporated herein by reference.

Especially favored in this invention is the formulation of the NACHE of Formula 1, into an orally-disintegrating tablet. A 40 to 60 mg tablet (using Ludipress® as the excipient), containing 1 to 5 mg of the NACHE, such as CPS-154, when tossed or placed in the back of the mouth rapidly exerts a robust anti-tussive action for several hours.

Dissolution of solid active ingredients in the oral cavity may sometimes be impeded by chewing or swallowing of the lozenge, or by the degree of hydration in the mouth.

A liquid formulation for delivery may therefore be preferable. The compounds of Formula 1 are readily soluble after warming in aqueous solutions containing polyhydric alcohols, cyclodextrins, sugars and the like. These liquids, after sterilization by filtration, may be combined with preservatives, flavoring agents, solvents, and then dispensed from a reservoir type of storage container (e.g., a plastic container with a dropper type of opening) or from unit dose containers such as are readily available commercially. For example, Unicep Corp. in Sandpoint, Idaho, USA, has unit-dose contract packaging methods for volumes of 0.3 to 0.5 mL. A 2 to 20 mg/mL dose of compounds of Formula 1 would be an ideal form of unit dosage at these volumes of delivery.

Examples of mouth spray delivery systems are currently marketed products such as Listerine Pocket Mist^{™} (Pfizer Consumer Healthcare) and Sweet Breath-breath spray (Health-Tech., Inc.). Examples of aerosol/nebulizer delivery systems are those systems marketed by Omron Healthcare, Inc., and A.D.A.M., Inc.

### Example 1

*N*-Acylated carboxamides can be synthesized by reacting a carboxylic acid with thionyl chloride and then conjugating the acid chloride to the appropriate amine. This is a chemical procedure familiar to the practitioners of the art and can be accomplished without undue experimentation.

In Formula 1, the carboxylic acid is (1*R*,2*S*,5*R*)-2-isopropyl-5-methyl-cyclohexanecarboxylic acid, the preferred enantiomer. This chemical is also known as WS-1 and may be synthesized from I-menthol or purchased from commercial firms. WS-1 is reacted with thionyl chloride to form the reagent p-menthoyl chloride, or it can be reacted directly *in situ* with the desired amine conjugate. The resulting product, usually a white crystalline solid, may then be purified by column chromatography and isolated by solvent evaporation. These are standard procedures of the organic chemistry laboratory.

### Synthesis of 2-Isopropyl-5-methyl-cyclohexanecarbonyl-D-Ala 3-hydroxypropyl ester (syn. (R)-2-[((1R,2S,5R)-2-isopropyl-5-methyl-cyclohexanecarbonyl)-amino]-propionic acid 3-hydroxy-propyl ester; p-Menthoyl-D-Ala-O-(CH₂)₃-OH):

4.8 g (25.5 mM) of Boc-D-Ala-OH, 1.26 g (10.3 mM) of DMAP (diaminopyridine) and an excess (30 mL) of 1,3-propanediol were dissolved in 30 mL of DMF (dimethylformamide) and the solution of 4.86 g (25.5 mM) of WSCD.HCl (1-ethyl-3-(3-dimethylaminopropyl)-carbodiimide hydrochloride) in 10 mL of DMF was stepwise added at 0°C with continuous stirring. The reaction mixture was stirred at 0°C for 1 hour and left overnight at ±4°C. The DMF was evaporated with n-butyl alcohol and water and the residue was diluted by ethyl acetate. The organic phase was washed by 5% NaHCO₃, water, 1 N H₂SO₄, water, dried by MgSO₄ and evaporated. For purification, a chromatography column (40 x 60 mm) with Kiselgel-60 (Merck) was used with elution by ethylacetate-hexane 1:3. Then, 3.7 g of the pure Boc-D-Ala-O-(CH₂)₃-OH was obtained (R_{f} = 0.63, CHCl₃ - MeOH - ethylacetate, 20:3:10). This product was treated with TFA (trifluoroacetic acid) for 30 minutes and the TFA was then evaporated. The TFA.H-D-Ala-O-(CH₂)₃-OH was then dissolved in DMF, the pH was adjusted to 8.5 by DIPEA and 3 mL (14.8 mM) of p-menthoylchloride and 2.6 mL (15 mM) of DIPEA were added stepwise to the reaction mixture with stirring at 0°C. The mixture was stirred for 1 hour at 0°C and 2 hours at room temperature. DMF was evaporated with n-butyl alcohol and water and the residue was diluted with ethyl acetate. The organic phase was washed by, 5% NaHCO₃, water, 1 N H₂SO₄, dried with MgSO₄ and evaporated. The product was purified on a LiChroprep RP-18 (30 x 100 mm) column with stepwise elution (30%, 40%, 50% i-PrOH/water). The product was crystallized in 25% i-PrOH/water, filtered off, and dried in vacuum. The product purity by FTPLC is 92%, and the yield was 1.8 g. The expected molecular mass was then confirmed by mass spectroscopy and the absorption spectrum by nuclear magnetic resonance. This compound was given the code of CPS-154.

For bioassay of CPS-154 on the skin, CPS-154 was stirred and dissolved in warm liquid Aquaphor® ointment to a yield ointment concentrations of 0.5 and 1% wt/vol. After cooling, 50 to 80 mg of the solid ointment was placed on the tip of a plastic stick and applied to the skin above the upper lip, on the philtrum, and lateral to the philtrum up to the nasolabial folds, of test subjects and the onset and duration of cooling sensations noted. The intensity of the subjective skin sensation was rated as 0, 1, 2 or 3 with 0 as no change, 1 as slight coolness, cold, or tingling, 2 as clear cut signal of coolness, cold, or tingling, and 3 as robust cooling or cold. The interval for recording sensations was at 5 minute intervals, until two successive zeroes were obtained. The results were averaged values of 4 to 6 separate trials in the same individual. The data were plotted using SigmaPlot (Systat Software, Point Richmond CA) and a smoothing function with a negative exponential was used for analysis and statistical fit of the results. The results for different compounds are shown in Figure 1, and, for comparative purposes, include compounds, shown in Table 1, with alkyl instead of hydroxyalkyl esters, and with L-amino acids instead of D-amino acids. CPS-154 produces cooling on the skin of the upper lip with duration of action of more than 1 hour.

Figure 1 illustrates the data for various compounds tested on the skin of the upper lip for cooling activities. The potency of CPS-154, a NACHE, was comparable to its alkyl ester analog, CPS-369. The L-amino acid analog of CPS-154, CPS-159, was less active. For comparison, the activity of WS-5 is shown. The introduction of a hydroxyalkyl group into the ester function results a compound with desirable properties.

### Example 2

The ADME parameters for these chemicals were computed using the ADMET Predictor^{™} and Modeler^{™} from Simulation-Plus, Lancaster, California. It can be seen that the NACHE compounds have water-solubility in the range of 2.27 to 3.14. From the analysis, it can be seen that the addition of a hydroxyalkyl to the ester group increases water solubility, lowers log P and reduces the likelihood of passage of the molecule across the blood-brain barrier.

| Table 1 | | | | | | |
|---|---|---|---|---|---|---|
| Properties of NACHE and Related Compounds | | | | | | |
| 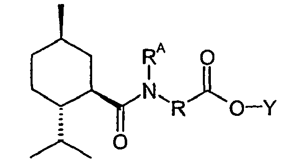 | | | | | | |

| Code | R^{A} | R | Y | LogP | Water solubility | BBB entry |
|---|---|---|---|---|---|---|
| WS-31 | -H | -CH₂- | -CH₃ | 2.66 | 0.44 | high |
| WS-5 | -H | -CH₂- | -CH₂-CH₃ | 3.07 | 0.21 | high |
| CPS-368 | -H | -CH(CH₃)- * | -CH₃ | 3.12 | 0.20 | high |
| CPS-337 | -H | -CH₂- | -CH₂-CH₃ | 3.39 | 0.29 | high |
| CPS-338 | -H | -CH₂-CH₂- | -CH₂-CH₃ | 3.40 | 0.12 | high |
| CPS-369 | -H | -CH(CH₃)- * | -CH₂-CH₃ | 3.76 | 0.07 | high |
| | | | | | | |
| CPS-153 | -H | -CH(CH₃)- * | -CH₂-CH₂OH | 2.27 | 0.68 | low |
| CPS-154 | -H | -CH(CH₃)- * | -CH₂-CH₂-CH₂OH | 2.67 | 0.75 | low |
| CPS-155 | -H | -CH(CH₃)- * | -CH₂-CH₂-CHOH-CH₃ | 3.12 | 0.82 | low |
| CPS-156 | -H | -CH(CH₃)- * | -CH₂-(CH₂)₂-CH₂OH | 3.14 | 0.82 | low |
| CPS-159 | -H | -CH(CH₃)- | -CH₂-CH₂-CH₂OH | 2.67 | 0.75 | low |

| | | | | | | |
|---|---|---|---|---|---|---|
| * denotes α-carbon is in the D-configuration. | | | | | | |

In the above table, BBB denotes blood-brain-barrier; water solubility is quoted in units of mol/L; and log P = log octanol/water partition coefficient. These parameters were obtained using a commercial computer analysis program for ADMET prediction.

### Example 3

Tablets of CPS-154 were prepared. To weighed samples of about 200 mg of CPS-154 was added varying amounts of Ludipress® (BASF, Germany) to give CPS-154 concentrations of 2.5, 3. 5, 10 and 10% by wt. The two ingredients were then thoroughly mixed in a mortar and pestle. To this mixture when then added 5 to 7 mL of 10% ethanol - 90% distilled water solution. Using an eye dropper or a pipette, fixed volumes of the solution was placed on wax paper and dried under a heat source. The tablets were then sized and weighed. When placed in the back of the mouth, these tablets dissolved within a matter of seconds and the active ingredient, CPS-154, could be felt in the pharynx.

Alternatively, these tablets may be manufactured using a rotary tablet compressor, wherein the API and excipient is mixed and then compressed (for example, at a force of 50 Newtons or less) in a die, using methods known to those skilled in the art.

Using the above formulation methods, three doses of 1, 2 and 4 mg per application were examined. At the 2 or 4 mg dose, CPS-154 tablets produced pleasant and robust cooling sensations on the tongue, extending down to the back of throat, and lasted for 20 to 30 minutes and longer. The onset effect was usually within 30 seconds of application. The D-analog was clearly more active than the L-equivalent, CPS-159. Menthol tested under the same conditions produced cooling primarily in the nasopharynx and less in the oral cavity. Also, the effects of menthol were short-lived, on the order 8 to 12 minutes, versus a 20 to 30 minute period or longer. Surprisingly, the NACHE analogs, which include CPS-154, were devoid of any unpleasant tastes akin to those produced by (-) menthol.

### Example 4

Two males, both aged 60 years, were inveterate cigar smokers consuming 3 to 5 cigars per day. They both had frequent bouts of dry, non-productive, hacking coughs. At night, there were episodes of insomnia characterized by difficulties in breathing, an oppressive pressure on the chest and throat, and subsequent awakening with sensations of choking. During the day, a frequent and persistent sensation was that of tickling, irritative feelings in the back of the throat that led to coughing.

In 10 trials per subject, 2 mg of 92% pure powder of CPS-154 was placed on the mid-portion of the tongue of these two subjects. Both subjects reported relief from the desire to cough within 1 minute after application. Sensations of robust coolness in the back of the throat was noted and lasted for 15 to 20 minutes. Applications of similar amounts of powdered sugar were not effective, but the placebo could be clearly distinguished from the active compound because of the absence of cooling sensations.

After application of test substance, the absence of irritation and itching at the back of the throat (cough signals) lasted for about 4 to 5 hours and the individual was observed not to cough for 6 to 8 hours. Surprisingly, after 8 applications in both subjects, the individuals noted that the desire to cough was removed for at least 3 days, in spite of continued smoking of cigars. This suggested that the test compound may have down-regulated the cough receptor mechanisms and permanently raised the threshold for cough stimuli. This therapeutic effect occurred when the test substance no longer elicited cooling sensations in the oropharynx. The CPS-154 is now also adapted for use as a mouth spray.

### Example 5

Ms. "Alice", aged 61 years, with a documented 31-year history of asthma attacks volunteered to test one of the compounds of Formula 1. She was extremely sensitive to agents such as tobacco smoke, dust, pollen, odors, down/woolen materials, house cleansing products, pets, and sudden change of air temperature and reacted with violent coughing, mucus secretions, wheezing, choking, and shortness of breath. At the time of this trial, she was taking these medications for her condition: theophylline, Allegra®, Advair®, Intal®, and Singulair®. She was given 20 tablets of CPS-154, synthesized as described in Example 3, and instructed to take one on an as-needed basis.

During a coughing fit, she took a tablet and reported a soothing and cool sensation with a slight taste similar to Eucalyptus oil in her mouth and throat. Coughing stopped after 3 to 5 minutes and breathing was normal. She did not sense any build up of sputum or other side-effects. In several trials when the compound was applied at night, she slept through the night without being awakened by cough. She now continues to use the test compound for management of her asthma, with the knowledge and consent of her physician. Surprisingly, her asthmatic coughing attacks have not recurred with any severity for six months. This effect suggests that one of the pathophysiological signs of asthma, airway hyper-reactivity, may be attenuated by the treatment.

### Example 6

Ms. "Janice", aged 60 years, has severe allergies and also developed a cough after a cruise trip overseas. Upon visit to a physician, she was diagnosed with a "viral cough" and prescribed codeine in a syrup. She volunteered to try CPS-154 tablets and was supplied with 15 tablets stored in a flip top plastic box, each containing 2.0 mg of CPS-154, in a 4% wt/wt lactose matrix (Ludipress®). The subject was instructed to take one tablet on an as-needed basis, with a preferred interval of 3 to 4 hours between tablets.

The subject reported the greatest benefit the first night after taking a tablet, as she was coughing frequently and could not go to sleep. The tablet enabled to fall asleep and rest for the whole night. She said she may have been excessively tired and that is why the tablet worked so well. On the next three days, she continued to cough and to take the tablet. The soothing effect of CPS-154 tablets was noticeable in that the subject, who likes to talk, found she was able to talk more without coughing. But when she was active in the daytime, she noted that the soothing effect lasted more like 1 to 2 hours instead of longer. There was no sense of inability to cough when there was perceived obstruction. In the presence of CPS-154, the throat was soothed, and the cough previously difficult to tolerate, was less severe. Her husband, who slept in the same bedroom, kept telling her to "take the magic pill" when she coughed.

In summary, the inventor has found that introduction of a hydroxyalkyl function into the ester group of a molecular pharmacophore of a cooling agent has the beneficial effect of cooling sensations without adverse effects on taste. Simulation of the structure also shows that this functional group reduces the likelihood of the molecule crossing the blood-brain-barrier. This insight into the molecular parameters of agonist action are utilized for the selection of ideal drug candidates for the treatment of cough and for disorders of the respiratory tree such as a cough tablet or a mouth spray.

The foregoing has described the principles, preferred embodiments, and modes of operation of the present invention. However, the invention should not be construed as limited to the particular embodiments discussed. Instead, the above-described embodiments should be regarded as illustrative rather than restrictive, and it should be appreciated that variations may be made in those embodiments by workers skilled in the art without departing from the scope of the present invention.

## Claims

1. A compound having the structure of Formula 1: 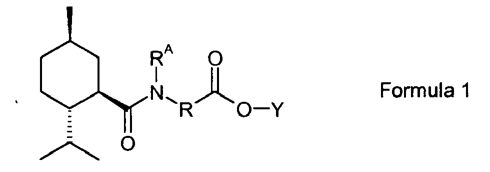 wherein:
R^{A} is -H or -CH₃;
R is a branched C₂-C₄ alkylidene, with the α-carbon in the D-configuration; and
Y is C₂-C₅ hydroxyalkyl.

2. A compound according to claim 1, wherein the compound of Formula 1 is (*R*)-2-[((*R*,2*S*,5*R*)-2-isopropyl-5-methyl-cyclohexanecarbonyl)-amino]-propionic acid 3-hydroxy-propyl ester.

3. A compound according to claim 1, wherein the compound of Formula 1 is (*R*)-2-[((1*R*,2*S*,5*R*)-2-isopropyl-5-methyl-cyclohexanecarbonyl)-amino]-propionic acid 2-hydroxy-1-methyl-propyl ester.

4. A composition comprising a compound having the structure of Formula 1: 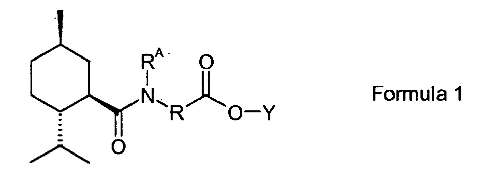 wherein:
R^{A} is -H or -CH₃;
R is a branched C₂-C₄ alkylidene, with the α-carbon in the D-configuration; and
Y is C₂-C₅ hydroxyalkyl.

5. A composition according to claim 4, wherein the compound of Formula 1 is (*R*)-2-[((1*R*,2*S*,5*R*)-2-isopropyl-5-methyl-cyclohexanecarbonyl)-amino]-propionic acid 3-hydroxy-propyl ester.

6. A composition according to claim 4, wherein the compound of Formula 1 is (*R*)-2-[((1*R*,2*S*,5*R*)-2-isopropyl-5-methyl-cyclohexanecarbonyl)-amino]-propionic acid 2-hydroxy-1-methyl-propyl ester.

7. A composition according to any one of claims 4 to 6, in the form of a mouth spray adapted to deliver the compound into the oral cavity and pharynx.

8. A composition according to any one of claims 4 to 6, in the form of an aerosol formulation adapted to deliver the compound into the respiratory tree.

9. A composition according to any one of claims 4 to 6, wherein the compound is further admixed with an agent selected from: an anti-inflammatory glucocorticosteroid, a sympathomimetic amine decongestant, a β₂-adrenergic receptor stimulant, an anti-histamine, a local anesthetic, menthol or a menthol analog, and mixtures thereof.

10. A compound according to any one of claims 1 to 3 or a composition according to any one of claims 4 to 6 for use in a method of treatment of the human or animal body by therapy.

11. A compound according to any one of claims 1 to 3 or a composition according to any one of claims 4 to 6 for use in a method of treatment of a respiratory disorder, an upper respiratory disorder, cough, asthma, or a chronic pulmonary disease.

12. A compound according to any one of claims 1 to 3 or a composition according to any one of claims 4 to 6 for use in a method of treatment of cough or throat irritation.

13. A compound according to any one of claims 1 to 3 or a composition according to any one of claims 4 to 6 for use as an anti-irritant.

14. Use of a compound according to any one of claims 1 to 3 or a composition according to any one of claims 4 to 6 in the manufacture of a medicament for the treatment of a respiratory disorder, an upper respiratory disorder, cough, asthma, or a chronic pulmonary disease.

15. Use of a compound according to any one of claims 1 to 3 or a composition according to any one of claims 4 to 6 in the manufacture of a medicament for the treatment of cough or throat irritation.

## Patentansprüche

1. Verbindung mit der Struktur der Formel (1): 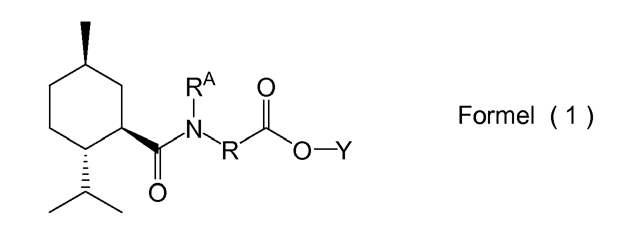 worin:
R^{a} -H oder -CH₃ ist;
R verzweigtes C₂₋₄-Alkyliden mit dem α-Kohlenstoff in der D-Konfiguration ist; und
Y C₂₋₅-Hydroxyalkyl ist.

2. Verbindung gemäss Anspruch 1, wobei die Verbindung der Formel (1) (R)-2-[((1R,2S,5R)-2-Isopropyl-5-methyl-cyclohexancarbonyl)-amino]-propionsäure-3-hydroxypropylester ist.

3. Verbindung gemäss Anspruch 1, wobei die Verbindung der Formel (1) (R)-2-[((1R,2S,5R)-2-Isopropyl-5-methyl-cyclohexancarbonyl)-amino]-propionsäure-2-hydroxy-1-methylpropylester ist.

4. Zusammensetzung, umfassend eine Verbindung mit der Struktur der Formel (1): 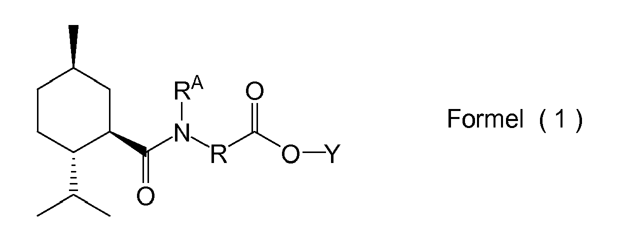 worin:
R^{a} -H oder -CH₃ ist;
R verzweigtes C₂₋₄-Alkyliden mit dem α-Kohlenstoff in der D-Konfiguration ist; und
Y C₂₋₅-Hydroxyalkyl ist.

5. Zusammensetzung gemäss Anspruch 4, wobei die Verbindung der Formel (1) (R)-2-[((1R,2S,5R)-2-Isopropyl-5-methyl-cyclohexancarbonyl)-amino]-propionsäure-3-hydroxypropylester ist.

6. Zusammensetzung gemäss Anspruch 4, wobei die Verbindung der Formel (1) (R)-2-[((1R,2S,5R)-2-Isopropyl-5-methyl-cyclohexancarbonyl)-amino]-propionsäure-2-hydroxy-1-methylpropylester ist.

7. Zusammensetzung gemäss irgendeinem der Ansprüche 4 bis 6 in Form eines Mundsprays, das zur Abgabe der Verbindung in die Mundhöhle und den Rachen geeignet ist.

8. Zusammensetzung gemäss irgendeinem der Ansprüche 4 bis 6 in Form einer Aerosolformulierung, die zur Abgabe der Verbindung in den Bronchialbaum geeignet ist.

9. Zusammensetzung gemäss irgendeinem der Ansprüche 4 bis 6, wobei die Verbindung ferner mit einem Mittel, ausgewählt aus einem entzündungshemmenden Glucocorticosteroid, einem sympathomimetischen Amin-Dekongestivum, einem β2-adrenergischen Rezeptorstimulans, einem Antihistamin, einem Lokalanästhetikum, Menthol oder einem Mentholanalogon und Mischungen davon, vermischt ist.

10. Verbindung gemäss irgendeinem der Ansprüche 1 bis 3 oder Zusammensetzung gemäss irgendeinem der Ansprüche 4 bis 6 zur Verwendung in einem Verfahren zur therapeutischen Behandlung des menschlichen oder tierischen Körpers.

11. Verbindung gemäss irgendeinem der Ansprüche 1 bis 3 oder Zusammensetzung gemäss irgendeinem der Ansprüche 4 bis 6 zur Verwendung in einem Verfahren zur Behandlung von Atemwegsstörung, einer Erkrankung der oberen Atemwege, Husten, Asthma oder einer chronischen Lungenerkrankung.

12. Verbindung gemäss irgendeinem der Ansprüche 1 bis 3 oder Zusammensetzung gemäss irgendeinem der Ansprüche 4 bis 6 zur Verwendung in einem Verfahren zur Behandlung von Husten oder einer Reizung des Rachens.

13. Verbindung gemäss irgendeinem der Ansprüche 1 bis 3 oder Zusammensetzung gemäss irgendeinem der Ansprüche 4 bis 6 zur Verwendung in einem Verfahren zur Behandlung zur Verwendung als Reizhemmer.

14. Verbindung gemäss irgendeinem der Ansprüche 1 bis 3 oder Zusammensetzung gemäss irgendeinem der Ansprüche 4 bis 6 zur Herstellung eines Medikaments zur Behandlung von Atemwegsstörung, einer Erkrankung der oberen Atemwege, Husten, Asthma oder einer chronischen Lungenerkrankung.

15. Verbindung gemäss irgendeinem der Ansprüche 1 bis 3 oder Zusammensetzung gemäss irgendeinem der Ansprüche 4 bis 6 zur Herstellung eines Medikaments zur Behandlung von Husten oder einer Reizung des Rachens.

## Revendications

1. Composé ayant la structure de formule 1 : 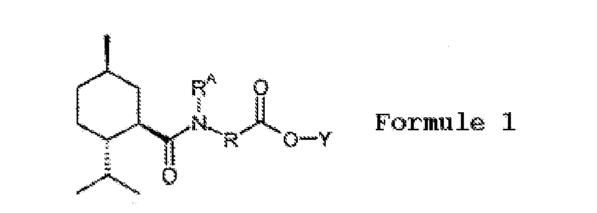 dans laquelle :
R^{A} représente -H ou -CH₃ ;
R représente un groupe alkylidène en C₂ à C₄, avec le carbone α dans la configuration D ; et
Y représente un groupe hydroxyalkyle en C₂ à C₅.

2. Composé selon la revendication 1, où le composé de formule 1 est l'ester 3-hydroxy-propylique de l'acide (*R*)-2-[((1*R*,2*S*,5*R*)-2-isopropyl-5-méthyl-cyclo-hexanecarbonyl)-amino]-propionique.

3. Composé selon la revendication 1, où le composé de formule 1 est l'ester 2-hydroxy-1-méthyl-propylique de l'acide (*R*)-2-[((1*R*,2*S*,5*R*)-2-isopropyl-5-méthyl-cyclohexanecarbonyl)-amino]-propionique.

4. Composition comprenant un composé ayant la structure de formule 1 : 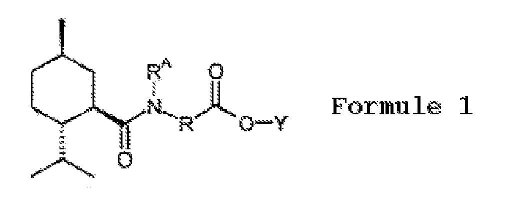 dans laquelle :
R^{A} représente -H ou -CH₃ ;
R représente un groupe alkylidène en C₂ à C₄, avec le carbone α dans la configuration D ; et
Y représente un groupe hydroxyalkyle en C₂ à C₅.

5. Composition selon la revendication 4, dans laquelle le composé de formule 1 est l'ester 3-hydroxy-propylique de l'acide (*R*)-2-[((1*R*,2*S*,5*R*)-2-isopropyl-5-méthyl-cyclohexanecarbonyl)-amino]-propionique.

6. Composition selon la revendication 4, dans laquelle le composé de formule 1 est l'ester 2-hydroxy-1-méthyl-propylique de l'acide (*R*)-2-[((1*R*,2*S*,5*R*)-2-isopropyl-5-méthyl-cyclohexanecarbonyl)-amino]-propionique.

7. Composition selon l'une quelconque des revendications 4 à 6, sous la forme d'un spray buccal adapté pour délivrer le composé dans la cavité orale et le pharynx.

8. Composition selon l'une quelconque des revendications 4 à 6, sous la forme d'une formulation d'aérosol adaptée pour délivrer le composé dans l'arbre respiratoire.

9. Composition selon l'une quelconque des revendications 4 à 6, dans laquelle le composé est en outre mélangé avec un agent choisi parmi : un anti-inflammatoire, un glucocorticoïde, un décongestionnant à base d'amine sympathomimétique, un stimulant du récepteur β₂-adrénergique, un antihistaminique, un anesthésique local, le menthol ou un analogue du menthol, et des mélanges de ceux-ci.

10. Composé selon l'une quelconque des revendications 1 à 3 ou composition selon l'une quelconque des revendications 4 à 6 en vue d'une utilisation dans un procédé de traitement du corps humain ou animal par une thérapie.

11. Composé selon l'une quelconque des revendications 1 à 3 ou composition selon l'une quelconque des revendications 4 à 6 en vue d'une utilisation dans un procédé de traitement d'un trouble respiratoire, d'un trouble respiratoire supérieur, de la toux, de l'asthme ou d'une maladie pulmonaire chronique.

12. Composé selon l'une quelconque des revendications 1 à 3 ou composition selon l'une quelconque des revendications 4 à 6 en vue d'une utilisation dans un procédé de traitement de la toux ou d'une irritation de la gorge.

13. Composé selon l'une quelconque des revendications 1 à 3 ou composition selon l'une quelconque des revendications 4 à 6 en vue d'une utilisation en tant qu'agent anti-irritant.

14. Utilisation d'un composé selon l'une quelconque des revendications 1 à 3 ou composition selon l'une quelconque des revendications 4 à 6 dans la fabrication d'un médicament destiné au traitement d'un trouble respiratoire, d'un trouble respiratoire supérieur, de la toux, de l'asthme ou d'une maladie pulmonaire chronique.

15. Utilisation d'un composé selon l'une quelconque des revendications 1 à 3 ou composition selon l'une quelconque des revendications 4 à 6 dans la fabrication d'un médicament destiné au traitement de la toux ou d'une irritation de la gorge.
